# EUROPEAN PATENT APPLICATION

(11) **EP 4 609 899 A1**
(43) Date of publication of application: **03.09.2025**
(21) Application number: 23882443.7
(22) Date of filing: 13.10.2023
(51) Int. Cl.: A61M 5/46, A61M 25/00, A61M 25/06

(54) **NEEDLE SYSTEM**

(30) Priority: 27.10.2022 JP 2022172403
(71) Applicant: KANEKA CORPORATION, Osaka 530-8288 (JP)
(72) Inventor: KOBAYASHI, Miho, Okaya-shi, Nagano 394-0034 (JP); MUKAI, Yuki, Okaya-shi, Nagano 394-0034 (JP)
(74) Representative: Hoffmann Eitle
(86) International application number: PCT/JP2023/037139
(87) International publication number: WO 2024/090235

(57) **Abstract**

Provided is a needle system (1) comprising: a resin tube (10) having an inner cavity (11) extending in a longitudinal direction (x) of the resin tube (10); a needle (20) formed from an electrically conductive material and disposed in the inner cavity (11) of the resin tube (10) so as to be movable in the longitudinal direction (x) with respect to the resin tube (10), the needle (20) being configured to puncture an organ inside a body; a first electrode (31) disposed on an outer surface (14) of the resin tube10; and a measurement device (40) configured to measure an electrical resistance between the needle (20) and the first electrode (31) when current is caused to flow between the needle (20) and the first electrode (31).

## Description

### TECHNICAL FIELD

The present invention relates to a needle system having a needle that punctures an organ inside a body.

### BACKGROUND ART

Therapies and the like that involve directly administering a drug solution such as a myocardial regenerative cell preparation to cardiac muscle cells that are losing the functions thereof owing to a myocardial infarction or the like so as to regenerate the cardiac muscle cells are performed. In a case where such a drug solution is directly administered to an organ inside a body in this manner, a catheter having a needle needs to be inserted into a body cavity, and the needle needs to be caused to puncture the organ. At this time, when a hole for administering the drug solution is not in a state of being located inside the organ owing to an excessively small depth of the puncture formed by the needle, the drug solution cannot be administered to the organ. In view of this, there has been a need to develop a needle that makes it possible to ascertain whether or not the needle has punctured the organ to such an extent that the hole for administering the drug solution is located inside the organ, so that the drug solution can be assuredly administered to the organ.

Patent Document 1 describes a drug solution injection needle which is a hollow needle for puncturing the myocardium of a patient and injecting a drug solution thereinto, the drug solution injection needle including: a sharp tip member formed from a metal; an electrically insulative connection tube connected to a proximal end side of the tip member; a metal tube connected to a proximal end side of the connection tube; and an insulating layer coating an outer circumferential surface of a proximal end portion of the metal tube. The connection tube and/or the tip member has an outer surface in which at least one hole (an outflow path for the drug solution) is opened, the at least one hole being formed in communication with an inner cavity of the needle. The metal tube has a distal end portion that is not coated with the insulating layer and that serves as an electrode for measuring a potential. This drug solution injection needle makes it possible to, by checking whether or not the potential measured by the electrode has a predetermined value or a larger value, easily determine whether or not an opening for injecting the drug solution is located inside a heart wall. Thus, an operation for injecting the drug solution is performed after it is determined that said potential has the predetermined value or a larger value, whereby the drug solution can be assuredly injected into the myocardium from the opening of the hole.

Patent Document 2 describes a drug solution injection needle system including: an electrode catheter including a distal end electrode; a drug solution injection needle; and a notification means. The drug solution injection needle includes: a sharp tip member formed from a metal; an electrically insulative connection tube connected to a proximal end side of the tip member; a metal tube connected to a proximal end side of the connection tube; and an insulating layer coating an outer circumferential surface of a proximal end portion of the metal tube. The connection tube and/or the tip member has an outer surface in which at least one hole is opened, the at least one hole being formed in communication with an inner cavity of the needle. The metal tube has a distal end portion that is not coated with the insulating layer and that serves as an electrode for measuring a potential. When a first potential measured by the distal end electrode has a first threshold value or a larger value and a second potential measured by the electrode of the drug solution injection needle has a second threshold value or a larger value, the notification means notifies an operator that a drug solution can be injected into the myocardium. This drug solution injection needle system performs an operation for injecting the drug solution after receiving a notification that is given from the notification means when the first potential measured by the distal end electrode of the electrode catheter has the first threshold value or a larger value and the second potential measured by the electrode of the drug solution injection needle has the second threshold value or a larger value. Therefore, the drug solution can be assuredly injected into the myocardium of the patient.

Patent Document 3 describes a drug solution injection needle which is a hollow needle for puncturing the myocardium of a patient and injecting a drug solution thereinto, the drug solution injection needle including: a first electrode which is for measuring a potential and which is a sharp metal member having a closed tip; an electrically insulative connection tube connected to a proximal end side of the first electrode; a metal tube connected to a proximal end side of the connection tube; and an insulating coating layer coating an outer circumferential surface of a proximal end portion of the metal tube. The connection tube has a tube wall through which at least one side hole (an outflow path for the drug solution) is opened in an outer circumferential surface of the connection tube, the at least one side hole being formed in communication with an inner cavity of the needle (an inner cavity of the connection tube). The metal tube has a distal end portion that is not coated with the insulating coating layer and that serves as a second electrode for measuring a potential. This drug solution injection needle makes it possible to, when both a potential measured by the first electrode and a potential measured by the second electrode have predetermined values or larger values, determine that an opening of the side hole formed through the tube wall of the connection tube is located inside the myocardium. Thus, an operation for injecting the drug solution is performed after it is determined that both potentials have the predetermined values or larger values, whereby the drug solution can be assuredly injected from the opening of the side hole into the myocardium.

### RELATED ART DOCUMENTS

### PATENT DOCUMENTS

Patent Document 1: International Publication No. WO2021/192283
Patent Document 2: International Publication No. WO2021/192284
Patent Document 3: International Publication No. WO2021/192285

### SUMMARY OF THE INVENTION

### TECHNICAL PROBLEM

However, the drug solution injection needles described in Patent Documents 1 to 3 have the following problem. Although each of the drug solution injection needles has an advantageous effect of making it possible to assuredly inject the drug solution into the myocardium through determination regarding whether or not the hole is present inside the cardiac muscle, the drug solution injection needle does not make it possible to ascertain the depth itself of the puncture formed by the needle, the depth indicating the length by which the needle has pierced the cardiac muscle. Thus, there is a possibility that the needle excessively punctures the cardiac muscle so that the needle penetrates and damages a tissue.

The present invention has been made in view of the aforementioned circumstances, and an object of the present invention is to provide a needle system that makes it possible to ascertain the depth by which a needle has punctured an organ inside a body.

### SOLUTIONS TO THE PROBLEMS

The gist of one embodiment of a needle system of the present invention is according to the present invention that can overcome the above problems is as follows.
[1] A needle system comprising:
   a resin tube having an inner cavity extending in a longitudinal direction of the resin tube;
   a needle formed from an electrically conductive material and disposed in the inner cavity of the resin tube so as to be movable in the longitudinal direction with respect to the resin tube, the needle being configured to puncture an organ inside a body;
   a first electrode disposed on an outer surface of the resin tube; and
   a measurement device configured to measure an electrical resistance between the needle and the first electrode when current is caused to flow between the needle and the first electrode.

The needle is transported to a treatment-target organ in a state of being present in the inner cavity of the resin tube. After the transport, the resin tube is caused to enter a state where a distal end portion thereof is in contact with the organ. Then, while current is kept flowing between the needle and the first electrode, the position of the needle with respect to the resin tube is shifted to the distal side such that the needle protrudes from the distal end portion of the resin tube so as to puncture the organ. By causing current to flow between the needle and the first electrode in a state where the distal end portion of the resin tube is in contact with the organ, the current flowing between the needle and the first electrode flows inside the organ. Consequently, the electrical resistance between the needle and the first electrode decreases at the moment when the needle punctures the organ. In addition, as the depth by which the needle has punctured the organ becomes larger, the contact area between the organ and the needle as an electricity flow path becomes larger, and thus the electrical resistance between the needle and the first electrode becomes lower. Therefore, by measuring the electrical resistance value between the needle and the first electrode when current is caused to flow between the needle and the first electrode, the depth by which the needle has punctured the organ can be ascertained according to the extent of a change in the value.

Preferred aspects of the needle system of the present invention are as shown in the following [2] to [11]:
[2] The needle system according to above [1], wherein the first electrode has an annular shape and is disposed on a distal portion of the resin tube.
[3] The needle system according to above [1] or [2], wherein
   the needle system further comprises a second electrode disposed on the outer surface of the resin tube so as to be located on a proximal side relative to the first electrode, and
   the measurement device is configured to measure an electrical resistance between the needle and the second electrode when current is caused to flow between the needle and the second electrode.
[4] The needle system according to above [3], wherein a gap of not shorter than a length of one said first electrode or one said second electrode in the longitudinal direction is present between the first electrode and the second electrode.
[5] The needle system according to above [1] to [4], wherein the needle has an inner cavity extending in the longitudinal direction.
[6] The needle system according to above [5], wherein the needle has, in a distal end thereof, an opening in communication with the inner cavity of the needle.
[7] The needle system according to above [5] or [6], wherein
   the needle has a side wall defining the inner cavity of the needle, and
   a hole in communication with the inner cavity of the needle is formed in the side wall of the needle within a distal portion of the needle.
[8] The needle system according to above [7], wherein
   a plurality of the holes are formed in the side wall of the needle, and
   the plurality of the holes are arranged in the longitudinal direction.
[9] The needle system according to above [1] to [8], wherein the needle is, over an entirety thereof, formed from a metal.
[10] The needle system according to above [1] to [9], wherein a first insulating base material is disposed on an outer surface, of the needle, that includes a distal end of the needle.
[11] The needle system according to above [10], wherein a second insulating base material is disposed on the outer surface of the needle so as to be located on a proximal side relative to a proximal end of the first insulating base material.

### EFFECTS OF THE INVENTION

The needle system according to the present invention measures the electrical resistance value between the needle and the first electrode when current is caused to flow between the needle and the first electrode, whereby the depth by which the needle has punctured the organ inside the body can be ascertained according to the extent of a change in the value.

### BRIEF DESCRIPTION OF THE DRAWINGS

[FIG. 1] FIG. 1 is a side view showing an example of the needle system according to the embodiment of the present invention.
[FIG. 2] FIG. 2 is a cross-sectional view (partially a side view) of the needle system shown in FIG. 1.
[FIG. 3] FIG. 3 is a cross-sectional view, of the needle system shown in FIG. 1, in which the distal portions of the resin tube and the needle are enlarged.
[FIG. 4] FIG. 4 is an end surface view of a cut portion at the line IV-IV shown in FIG. 3.
[FIG. 5] FIG. 5 is a cross-sectional view, of the needle system shown in FIG. 2, in which the distal portions of the resin tube and the needle are enlarged.
[FIG. 6] FIG. 6 is a side view (partially a cross-sectional view) showing a modification of the resin tube and the needle shown in FIG. 5.
[FIG. 7] FIG. 7 is a cross-sectional view of the resin tube and the needle shown in FIG. 6.
[FIG. 8] FIG. 8 is an end surface view of a cut portion at the line VIII-VIII shown in FIG. 6.
[FIG. 9] FIG. 9 is a side view (partially a cross-sectional view) showing another modification of the resin tube and the needle shown in FIG. 5.
[FIG. 10] FIG. 10 is a cross-sectional view of the resin tube and the needle shown in FIG. 9.
[FIG. 11] FIG. 11 is an end surface view of a cut portion at the line XI-XI shown in FIG. 9.
[FIG. 12] FIG. 12 is an end surface view of a cut portion at the line XII-XII shown in FIG. 9.
[FIG. 13] FIG. 13 is a side view (partially a cross-sectional view) showing still another modification of the resin tube and the needle shown in FIG. 5.
[FIG. 14] FIG. 14 is a cross-sectional view of the resin tube and the needle shown in FIG. 13.
[FIG. 15] FIG. 15 is an end surface view of a cut portion at the line XV-XV shown in FIG. 13.
[FIG. 16] FIG. 16 is an end surface view of a cut portion at the line XIV-XIV shown in FIG. 3.
[FIG. 17] FIG. 17 is a side view showing a modification of the needle system according to the embodiment of the present invention.
[FIG. 18] FIG. 18 is a cross-sectional view (partially a side view) of the needle system shown in FIG. 17.

### DESCRIPTION OF EMBODIMENTS

The present invention will be specifically explained below based on the following embodiments, however, the present invention is not restricted by the embodiments described below of course, and can be certainly put into practice after appropriate modifications within in a range meeting the gist of the above and the below, all of which are included in the technical scope of the present invention. In the drawings, hatching, a reference sign for a member may be omitted for convenience, and in such a case, the description and other drawings should be referred to. In addition, sizes of various members in the drawings may differ from the actual sizes thereof, since priority is given to understanding the features of the present invention.

A needle system according to an embodiment of the present invention has the following gist. That is, the needle system includes: a resin tube having an inner cavity extending in a longitudinal direction of the resin tube; a needle formed from an electrically conductive material and disposed in the inner cavity of the resin tube so as to be movable in the longitudinal direction of the resin tube with respect to the resin tube, the needle being configured to puncture an organ inside a body; a first electrode disposed on an outer surface of the resin tube; and a measurement device configured to measure an electrical resistance between the needle and the first electrode when current is caused to flow between the needle and the first electrode.

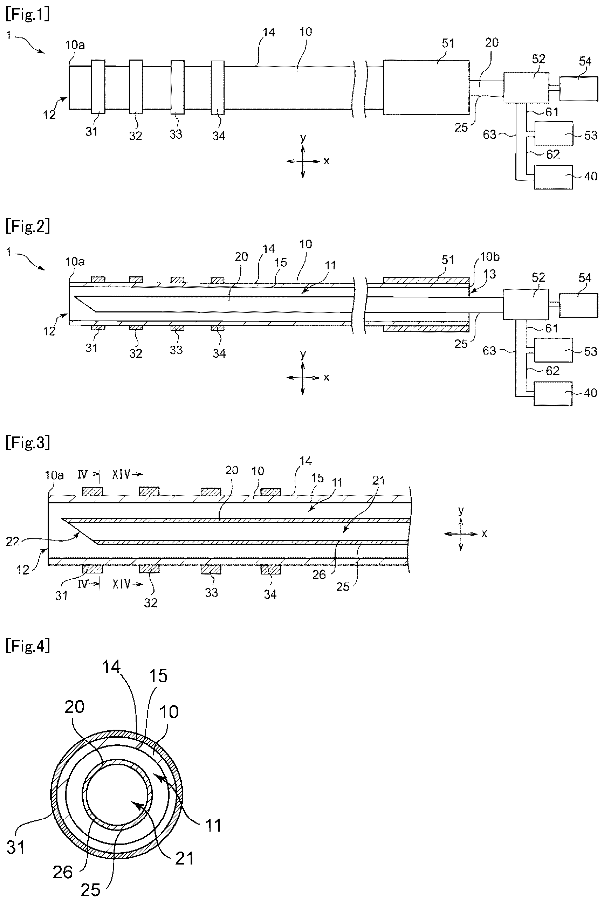

An overall configuration of the needle system according to the embodiment of the present invention will be described with reference to FIG. 1 to FIG. 18. FIG. 1 and FIG. 2 show a needle system 1 including a resin tube 10, a needle 20, a first electrode 31, and a measurement device 40. In these drawings, the longitudinal direction and the radial direction of the resin tube 10 are respectively indicated by x and y. The radial direction y is a direction perpendicular to the longitudinal direction x. The longitudinal direction x of the resin tube 10 is an extension direction of the resin tube 10.

In the present description, a proximal side refers to the hand side of a user in the extension direction of the resin tube 10, and a distal side refers to the side opposite to the proximal side, i.e., a treatment target side. A distal portion of each member refers to the distal half of the member, and a proximal portion of each member refers to the proximal half of the member.

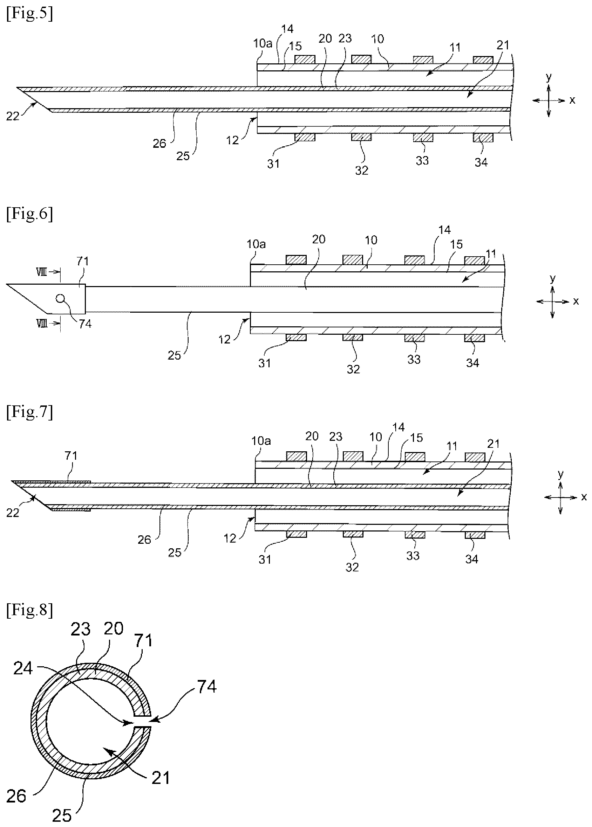

FIG. 1 is a side view showing an example of the needle system according to the embodiment of the present invention. FIG. 2 is a cross-sectional view (partially a side view) of the needle system shown in FIG. 1. FIG. 3 is a cross-sectional view, of the needle system shown in FIG. 1, in which the distal portions of the resin tube and the needle are enlarged. FIG. 4 is an end surface view of a cut portion at the line IV-IV shown in FIG. 3. FIG. 5 is a cross-sectional view, of the needle system shown in FIG. 2, in which the distal portions of the resin tube and the needle are enlarged.

As shown in FIG. 1 to FIG. 3, the needle system 1 includes the resin tube 10. The resin tube 10 has an inner cavity 11 extending in the longitudinal direction x. As shown in FIG. 2, the resin tube 10 may have a distal end 10a and a proximal end 10b. The resin tube 10 preferably has an outer surface 14 facing the outside of the resin tube 10 and an inner surface 15 facing the inner cavity 11.

As shown in FIG. 1 and FIG. 2, the needle system 1 includes the needle 20. The needle 20 is disposed in the inner cavity 11 of the resin tube 10 so as to be movable in the longitudinal direction x of the resin tube 10 with respect to the resin tube 10 and punctures an organ inside a body. The organ inside the body refers to an organ that is inside a body and, in particular, inside the abdomen or the thorax of the body. The organ is also called a viscus. Also, the needle 20 is formed from an electrically conductive material, and thus serves as an electrode. The needle 20 may be, over the entirety thereof or only partially, formed from an electrically conductive material.

As shown in FIG. 1 and FIG. 2, the needle system 1 includes the first electrode 31. The first electrode 31 is formed from an electrically conductive material and disposed on the outer surface 14 of the resin tube 10.

As shown in FIG. 1 and FIG. 2, the needle system 1 includes the measurement device 40. The measurement device 40 measures an electrical resistance between the needle 20 and the first electrode 31 when current is caused to flow between the needle 20 and the first electrode 31.

In the above needle system 1, the needle 20 is transported to a treatment-target organ in a state of being present in the inner cavity 11 of the resin tube 10 as shown in FIG. 1 to FIG. 3. After the transport, the resin tube 10 is caused to enter a state where a distal end portion thereof is in contact with the organ. Then, while current is kept flowing between the needle 20 and the first electrode 31, the position of the needle 20 with respect to the resin tube 10 is shifted to the distal side such that the needle 20 protrudes from the distal end portion of the resin tube 10 so as to puncture the organ. The state where the needle 20 protrudes from the distal end portion of the resin tube 10 is shown in FIG. 5. By causing current to flow between the needle 20 and the first electrode 31 in a state where the distal end portion of the resin tube 10 is in contact with the organ, the current flowing between the needle 20 and the first electrode 31 flows inside the organ. Consequently, the electrical resistance between the needle 20 and the first electrode 31 decreases at the moment when the needle 20 punctures the organ. In addition, as the depth by which the needle 20 has punctured the organ becomes larger, the contact area between the organ and the needle 20 as an electricity flow path becomes larger, and thus the electrical resistance between the needle 20 and the first electrode 31 becomes lower. Therefore, by measuring the electrical resistance value between the needle 20 and the first electrode 31 when current is caused to flow between the needle 20 and the first electrode 31, the depth by which the needle 20 has punctured the organ can be ascertained according to the extent of a change in the value.

The above needle system 1 can be used for directly administering a liquid such as a cell preparation or a drug solution to an organ, e.g., the heart, the kidney, the liver, or the like, inside a body. Specifically, the needle system 1 can be used for directly administering an iPS cell suspension to the liver or the kidney or directly administering a myocardial regenerative cell preparation to the heart, more specifically, the cardiac muscle.

The resin tube 10 may be produced through, for example, extrusion molding. The resin tube 10 may be composed of a single layer or a plurality of layers. The resin tube 10 may be such that: one portion thereof extending in the longitudinal direction x or the circumferential direction is composed of a single layer; and another portion thereof extending in the longitudinal direction x or the circumferential direction is composed of a plurality of layers.

The shape of the resin tube 10 is preferably a tubular shape and may be a shape such as the shape of a hollow column or a hollow polygonal prism.

The resin tube 10 may be formed from, for example, a synthetic resin such as a polyolefin resin (e.g., polyethylene or polypropylene), a polyamide resin (e.g., nylon), a polyester resin (e.g., PET), an aromatic polyether ketone resin (e.g., PEEK), a polyether polyamide resin, a polyurethane resin, a polyimide resin, or a fluorine resin (e.g., PTFE, PFA, or ETFE). These types of materials may be used singly, or two or more of these types of materials may be used in combination.

As shown in FIG. 1 and FIG. 2, a configuration may be employed in which a first handle 51 to be gripped by a user is connected to the proximal portion of the resin tube 10.

As shown in FIG. 2, the resin tube 10 may have a distal-side opening 12 and a proximal-side opening 13 which are in communication with the inner cavity 11.

At the time of bringing the distal end portion of the resin tube 10 into contact with the treatment-target organ after the needle 20 is transported to the organ in a state of being present in the inner cavity 11 of the resin tube 10, the contact is preferably performed such that the distal-side opening 12 of the resin tube 10 is closed by the organ.

The needle 20 can puncture an organ, e.g., the heart, the kidney, the liver, or the like, inside the body.

As shown in FIG. 1 and FIG. 2, a configuration may be employed in which a second handle 52 for adjusting the position of the needle 20 in the inner cavity 11 of the resin tube 10 is connected to the proximal portion of the needle 20.

As shown in FIG. 2, the needle 20 has a proximal end which is preferably located on the proximal side relative to the proximal end 10b of the resin tube 10. Consequently, adjustment of the position of the needle 20 in the inner cavity 11 of the resin tube 10 can be facilitated.

As shown in FIG. 2, it is preferable that, in the longitudinal direction x of the resin tube 10, a length of the needle 20 is longer than a length of the resin tube 10. Consequently, transmission of torques in the rotation direction in addition to the front-rear direction of pushing and pulling, i.e., motions in three-dimensional directions, to the needle 20 can be facilitated. Thus, adjustment of the position of the needle 20 in the inner cavity 11 of the resin tube 10 can be facilitated, and protrusion of the needle 20 from the distal end portion of the resin tube 10 can be facilitated. Therefore, puncturing of the organ inside the body by the needle 20 can be facilitated.

The needle 20 may be formed from a metal. The needle 20 may be, over the entirety thereof, formed from a metal. The needle 20 may be such that: only one portion thereof is formed from a metal; and another portion thereof is formed from a material other than metal, such as an electrically conductive resin.

Examples of the metal forming the needle 20 include stainless steels such as SUS304 and SUS316, platinum, nickel, cobalt, chromium, titanium, tungsten, gold, Ni-Ti alloys, Co-Cr alloys, or a combination of these metals.

The needle 20 may have a portion formed from a material having a larger electrical resistance value than a tissue to be punctured by the needle 20. The needle 20 may be, at only a portion thereof, formed from a material having a larger electrical resistance value than the tissue to be punctured by the needle 20. Consequently, the electricity flow amount decreases at the portion formed from the material having a larger electrical resistance value than the tissue to be punctured. Thus, a decrease in the electrical resistance is easily achieved when the needle 20 punctures the organ. Therefore, detection of a change in the electrical resistance value can be facilitated, whereby ascertainment of the depth by which the needle 20 has punctured the organ can be facilitated. Alternatively, the needle 20 may be, over the entirety thereof, formed from the material having a larger electrical resistance value than the tissue to be punctured by the needle 20. Consequently, increase of the extent of the change in the electrical resistance value between before and after the puncturing by the needle 20 can be facilitated, whereby ascertainment of the fact that the needle 20 has punctured the organ can be facilitated.

The shape of the needle 20 is preferably a tubular shape. For example, the shape may be a shape such as the shape of a hollow column or a hollow polygonal prism.

As shown in FIG. 3 and FIG. 4, a configuration may be employed in which the needle 20 has an inner cavity 21 extending in the longitudinal direction x of the resin tube 10. In this case, the needle 20 may have an outer surface 25 located on the resin tube 10 side and an inner surface 26 facing the inner cavity 21 of the needle 20. Consequently, a liquid such as a cell preparation or a drug solution can be injected into the inner cavity 21 of the needle 20.

As shown in FIG. 2, the needle system 1 may further include a liquid supply device 54 connected to the second handle 52. In this case, the needle system 1 is preferably configured to be able to supply the liquid such as a cell preparation or a drug solution from the liquid supply device 54 to the inner cavity 21 of the needle 20. For example, a configuration may be employed in which: the second handle 52 has an inner cavity; the inner cavity of the second handle 52 and the inner cavity 21 of the needle 20 are in communication with each other; and the liquid such as a cell preparation or a drug solution supplied from the liquid supply device 54 is supplied via the inner cavity of the second handle 52 to the inner cavity 21 of the needle 20. Although not shown, the supply of the liquid such as a cell preparation or a drug solution to the inner cavity 21 of the needle 20 may be achieved by directly connecting a proximal end portion of the needle 20 to the liquid supply device 54.

As the liquid supply device 54, for example, a device that is publicly known as a means for supplying a liquid and that is exemplified by a syringe or a pump may be used.

As shown in FIG. 3, a configuration may be employed in which the needle 20 has, in a distal end thereof, an opening 22 in communication with the inner cavity 21 of the needle 20. The opening 22 formed in the distal end of the needle 20 refers to an opening that can be seen when the needle 20 is observed in the longitudinal direction x of the resin tube 10. The opening 22 is preferably formed in a distal end surface of the needle 20. As shown in FIG. 3, the distal end surface of the needle 20 may be tilted with respect to the longitudinal direction x and the radial direction y of the resin tube 10, and the needle 20 may have the opening 22 in the tilted portion. Consequently, the liquid such as a cell preparation or a drug solution injected to the inner cavity 21 of the needle 20 can be discharged from the opening 22 formed in the distal end of the needle 20.

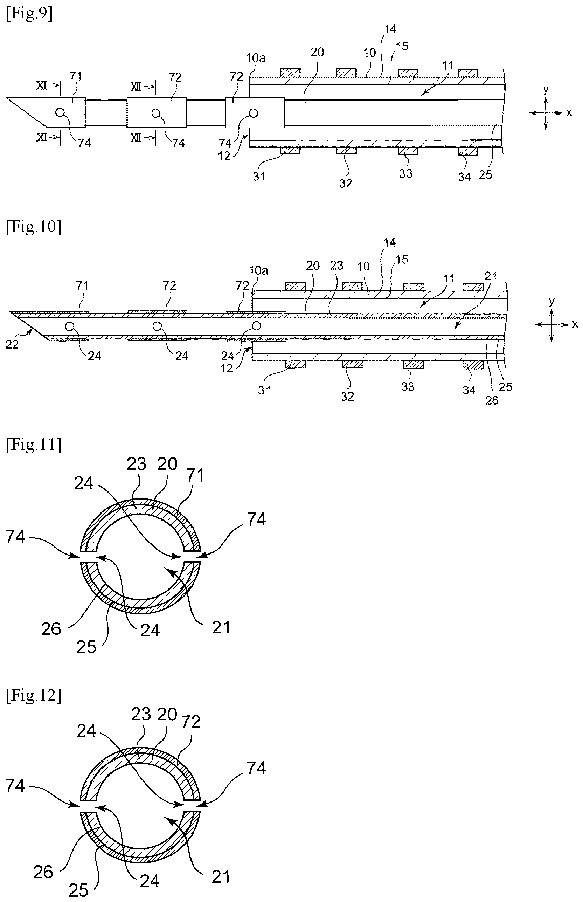

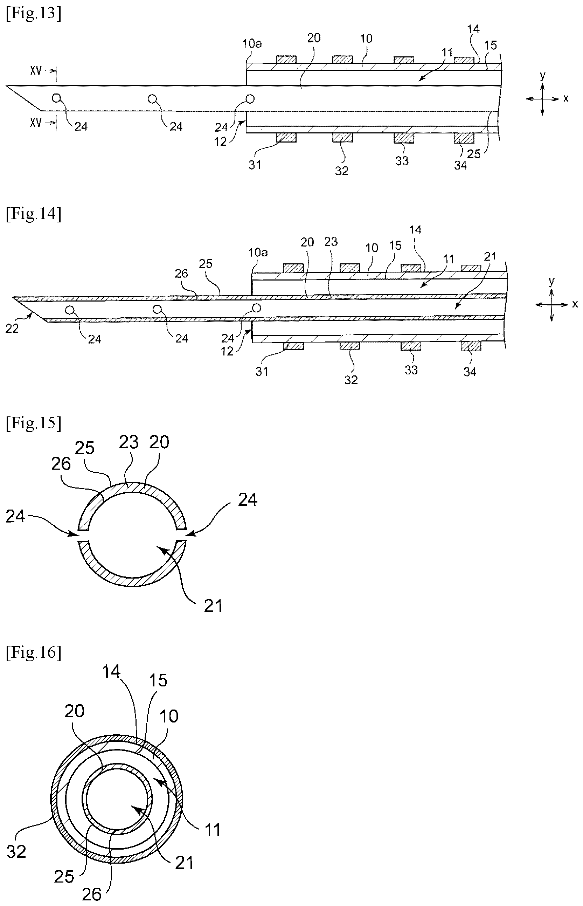

FIG. 6 is a side view (partially a cross-sectional view) showing a modification of the resin tube and the needle shown in FIG. 5. FIG. 7 is a cross-sectional view of the resin tube and the needle shown in FIG. 6. FIG. 8 is an end surface view of a cut portion at the line VIII-VIII shown in FIG. 6. FIG. 9 is a side view (partially a cross-sectional view) showing another modification of the resin tube and the needle shown in FIG. 5. FIG. 10 is a cross-sectional view of the resin tube and the needle shown in FIG. 9. FIG. 11 is an end surface view of a cut portion at the line XI-XI shown in FIG. 9. FIG. 12 is an end surface view of a cut portion at the line XII-XII shown in FIG. 9. FIG. 13 is a side view (partially a cross-sectional view) showing still another modification of the resin tube and the needle shown in FIG. 5. FIG. 14 is a cross-sectional view of the resin tube and the needle shown in FIG. 13. FIG. 15 is an end surface view of a cut portion at the line XV-XV shown in FIG. 13. FIG. 16 is an end surface view of a cut portion at the line XIV-XIV shown in FIG. 3.

As shown in FIG. 6 to FIG. 15, the needle 20 may have a side wall 23 defining the inner cavity 21 of the needle 20, and a hole 24 in communication with the inner cavity 21 of the needle 20 may be formed in the side wall 23 of the needle 20 within the distal portion of the needle 20. The hole 24 formed in the side wall 23 of the needle 20 refers to a hole that can be seen when the needle 20 is observed in the radial direction y of the resin tube 10. Employment of the above configuration causes the portion having the hole 24 formed therein to have a narrower electricity flow path, and thus causes said portion to have a smaller electricity flow amount. Thus, a decrease in the electrical resistance is easily achieved when the needle 20 punctures the organ. Therefore, detection of a change in the electrical resistance value can be facilitated, whereby ascertainment of the depth by which the needle 20 has punctured the organ can be facilitated. In addition, the liquid such as a cell preparation or a drug solution injected to the inner cavity 21 of the needle 20 can be discharged from the hole 24.

The number of the holes 24 to be formed in the needle 20 is not particularly limited and, for example, may be only one as shown in FIG. 6 to FIG. 8 or may be two or more as shown in FIG. 9 to FIG. 15.

The shape of the hole 24 to be formed in the needle 20 is not particularly limited and, for example, may be a circular shape, an elliptical shape, a polygonal shape, or the like. From the viewpoint of ease of formation of the hole 24, the shape is preferably a circular shape.

As shown in FIG. 9 to FIG. 15, a plurality of the holes 24 may be formed in the side wall 23 of the needle 20, and the plurality of the holes 24 may be arranged in the longitudinal direction x of the resin tube 10. All of the plurality of the holes 24 may be arranged in the longitudinal direction x of the resin tube 10. Employment of this configuration causes the portion having the holes 24 formed therein to have a narrower electricity flow path, and thus causes said portion to have a smaller electricity flow amount. Thus, a decrease in the electrical resistance is easily achieved when the needle 20 punctures the organ. Therefore, detection of a change in the electrical resistance value can be facilitated, whereby ascertainment of the depth by which the needle 20 has punctured the organ can be facilitated. In addition, the liquid such as a cell preparation or a drug solution injected to the inner cavity 21 of the needle 20 can be discharged from the plurality of the holes 24, whereby the liquid such as a cell preparation or a drug solution can be efficiently injected.

As shown in FIG. 1 to FIG. 5, a configuration may be employed in which the needle 20 only has the opening 22 formed in the distal end thereof and does not have any hole 24 formed in the side wall 23. Although not shown, a configuration may be employed in which the needle 20 only has the hole 24 formed in the side wall 23 and does not have any opening 22 formed in the distal end thereof. As shown in FIG. 6 to FIG. 15, the needle 20 may have both the opening 22 formed in the distal end of the needle 20 and the hole 24 formed in the side wall 23 of the needle 20.

As shown in FIG. 6 to FIG. 11, a first insulating base material 71 may be disposed on the outer surface 25, of the needle 20, that includes the distal end of the needle 20. Employment of this configuration, the portion where the first insulating base material 71 is disposed prevents a contact area where the needle 20 is in contact with the organ from increasing, and this prevents an electricity flow path between the needle 20 and the organ from increasing. Thus, the electricity flow amount is kept unchanged while puncturing is being performed no deeper than the portion on which the first insulating base material 71 is disposed. Consequently, it becomes easy to ascertain a decrease in the electrical resistance when puncturing by the needle 20 has been performed as deep as a portion on which the first insulating base material 71 is not disposed. Therefore, detection of a change in the electrical resistance value can be facilitated, whereby ascertainment of the depth by which the needle 20 has punctured the organ can be facilitated.

As shown in FIG. 9 to FIG. 12, a second insulating base material 72 may be disposed on the outer surface 25 of the needle 20 so as to be located on the proximal side relative to a proximal end of the first insulating base material 71. Employment of this configuration, the portion where the second insulating base material 72 is disposed prevents a contact area where the needle 20 is in contact with the organ from increasing, and this prevents an electricity flow path between the needle 20 and the organ from increasing. Thus, the electricity flow amount is kept unchanged while the portion, on which the second insulating base material 72 is disposed, is newly entering the organ. Consequently, it becomes easy to ascertain a decrease in the electrical resistance when puncturing by the needle 20 has been performed as deep as a portion on which the second insulating base material 72 is not disposed. Therefore, detection of a change in the electrical resistance value can be facilitated, whereby ascertainment of the depth by which the needle 20 has punctured the organ can be facilitated.

The first insulating base material 71 and the second insulating base material 72 are members having characteristics of hardly conducting electricity.

The first insulating base material 71 and the second insulating base material 72 are preferably formed from resins. Examples of the resins forming the first insulating base material 71 and the second insulating base material 72 can include polyimide resins, polyamide resins, polyolefin resins, polyester resins, polycarbonate resins, epoxy resins, and the like. The resins forming the first insulating base material 71 and the second insulating base material 72 may be identical to or different from each other.

As shown in FIG. 9 and FIG. 10, a plurality of the second insulating base materials 72 may be disposed on the outer surface 25 of the needle 20. Although not shown, one said second insulating base material 72 may be disposed alone on the outer surface 25 of the needle 20.

As shown in FIG. 6, the first insulating base material 71 is located on only a part of the outer surface 25 of the needle 20 and is configured not to cover the entirety of the outer surface 25 of the needle 20. As shown in FIG. 9, the second insulating base materials 72 are located on only parts of the outer surface 25 of the needle 20 and are configured not to cover the entirety of the outer surface 25 of the needle 20. As shown in FIG. 9, the first insulating base material 71 and the second insulating base materials 72 are located on only parts of the outer surface 25 of the needle 20 and are configured not to cover the entirety of the outer surface 25 of the needle 20.

As shown in FIG. 6 to FIG. 12, a configuration is preferably employed in which: the first insulating base material 71 and the second insulating base materials 72 are formed in tubular shapes; and the needle 20 is disposed in inner cavities of the first insulating base material 71 and the second insulating base materials 72 formed in tubular shapes.

As shown in FIG. 6 to FIG. 12, in a case where each of the holes 24 is formed in a portion, of the needle 20, on which a corresponding one of the first insulating base material 71 or the second insulating base materials 72 is disposed, a hole 74 in communication with the hole 24 of the needle 20 is preferably formed in the first insulating base material 71 or the second insulating base material 72. Although not shown, the hole 24 may be formed in a portion, of the needle 20, on which neither the first insulating base material 71 nor the second insulating base material 72 is disposed.

As shown in FIG. 13 to FIG. 15, each hole 24 may be formed in a needle 20 on which neither the first insulating base material 71 nor the second insulating base material 72 is disposed.

As shown in FIG. 1 to FIG. 3, the first electrode 31 preferably has an annular shape or a tubular shape. The first electrode 31 is preferably disposed on the distal portion of the resin tube 10 and is more preferably disposed on the distal portion of the resin tube 10 so as to be located on the proximal side relative to the distal end of the resin tube 10.

The first electrode 31 is preferably connected to the measurement device 40 described later. The first electrode 31 does not have to be connected to a display device 41 described later.

As long as the measurement device 40 can measure the electrical resistance between the needle 20 and the first electrode 31, the measurement device 40 is not particularly limited, and a publicly known measurement device is usable. The measurement device 40 is preferably connected to the needle 20 and the first electrode 31.

As shown in FIG. 1 and FIG. 2, the needle system 1 may further include a second electrode 32 disposed on the outer surface 14 of the resin tube 10 so as to be located on the proximal side relative to the first electrode 31. The needle system 1 may further include a third electrode 33 disposed on the outer surface 14 of the resin tube 10 so as to be located on the proximal side relative to the second electrode 32. The needle system 1 may further include a fourth electrode 34 disposed on the outer surface 14 of the resin tube 10 so as to be located on the proximal side relative to the third electrode 33. Each of the second electrode 32, the third electrode 33, and the fourth electrode 34 is preferably disposed on the distal portion of the resin tube 10.

As shown in FIG. 1 and FIG. 2, it is preferable that: the needle system 1 further includes the second electrode 32 disposed on the outer surface 14 of the resin tube 10 so as to be located on the proximal side relative to the first electrode 31; and the measurement device 40 is configured to measure an electrical resistance between the needle 20 and the second electrode 32 when current is caused to flow between the needle 20 and the second electrode 32. The second electrode 32 is preferably connected to the measurement device 40. By causing current to flow between the needle 20 and the second electrode 32 in a state where the distal end portion of the resin tube 10 is in contact with the organ, the current flowing between the needle 20 and the second electrode 32 flows inside the organ. Consequently, the electrical resistance between the needle 20 and the second electrode 32 decreases at the moment when the needle 20 punctures the organ. In addition, as the depth by which the needle 20 has punctured the organ becomes larger, the contact area between the organ and the needle 20 as an electricity flow path becomes larger, and thus the electrical resistance between the needle 20 and the second electrode 32 becomes lower. Therefore, by measuring the electrical resistance value between the needle 20 and the second electrode 32 when current is caused to flow between the needle 20 and the second electrode 32, the depth by which the needle 20 has punctured the organ can be ascertained according to the extent of a change in the value. This aspect is suitably applicable in a case where, for example, the first electrode 31 is in contact with the cardiac muscle, and the second electrode 32 is not in contact with the cardiac muscle. The electrical resistance can be measured also in a case where the first electrode 31 is in contact with the cardiac muscle. However, since the cardiac muscle has a higher electrical resistance than blood, the difference in the electrical resistance when the needle 20 has pierced the cardiac muscle from a state of being present in blood is larger so that it is easier to ascertain that the needle 20 has pierced the cardiac muscle, in a case where the electrode is not in contact with the cardiac muscle than in a case where the electrode is in contact with the cardiac muscle. In this manner, improvement of the measurement accuracy can be more facilitated in a state where the electrode is not in contact with the cardiac muscle at all, i.e., in a state where the entirety of the electrode is in contact with blood, than in a state where the electrode is in contact with the cardiac muscle.

As shown in FIG. 1 and FIG. 2, it is preferable that: the needle system 1 further includes the third electrode 33 disposed on the outer surface 14 of the resin tube 10 so as to be located on the proximal side relative to the second electrode 32; and the measurement device 40 is configured to measure an electrical resistance between the needle 20 and the third electrode 33 when current is caused to flow between the needle 20 and the third electrode 33. The third electrode 33 is preferably connected to the measurement device 40. By causing current to flow between the needle 20 and the third electrode 33 in a state where the distal end portion of the resin tube 10 is in contact with the organ, the current flowing between the needle 20 and the third electrode 33 flows inside the organ. Consequently, the electrical resistance between the needle 20 and the third electrode 33 decreases at the moment when the needle 20 punctures the organ. In addition, as the depth by which the needle 20 has punctured the organ becomes larger, the contact area between the organ and the needle 20 as an electricity flow path becomes larger, and thus the electrical resistance between the needle 20 and the third electrode 33 becomes lower. Therefore, by measuring the electrical resistance value between the needle 20 and the third electrode 33 when current is caused to flow between the needle 20 and the third electrode 33, the depth by which the needle 20 has punctured the organ can be ascertained according to the extent of a change in the value. This aspect is suitably applicable in a case where, for example, the first electrode 31 and the second electrode 32 are in contact with the cardiac muscle, and the third electrode 33 is not in contact with the cardiac muscle. The electrical resistance can be measured also in a case where each of the first electrode 31 and the second electrode 32 is in contact with the cardiac muscle. However, since the cardiac muscle has a higher electrical resistance than blood, the difference in the electrical resistance when the needle 20 has pierced the cardiac muscle from a state of being present in blood is larger so that it is easier to ascertain that the needle 20 has pierced the cardiac muscle, in a case where the electrode is not in contact with the cardiac muscle than in a case where the electrode is in contact with the cardiac muscle. In this manner, improvement of the measurement accuracy can be more facilitated in a state where the electrode is not in contact with the cardiac muscle at all, i.e., in a state where the entirety of the electrode is in contact with blood, than in a state where the electrode is in contact with the cardiac muscle.

As shown in FIG. 1 and FIG. 2, it is preferable that: the needle system 1 further includes the fourth electrode 34 disposed on the outer surface 14 of the resin tube 10 so as to be located on the proximal side relative to the third electrode 33; and the measurement device 40 is configured to measure an electrical resistance between the needle 20 and the fourth electrode 34 when current is caused to flow between the needle 20 and the fourth electrode 34. The fourth electrode 34 is preferably connected to the measurement device 40. By causing current to flow between the needle 20 and the fourth electrode 34 in a state where the distal end portion of the resin tube 10 is in contact with the organ, the current flowing between the needle 20 and the fourth electrode 34 flows inside the organ. Consequently, the electrical resistance between the needle 20 and the fourth electrode 34 decreases at the moment when the needle 20 punctures the organ. In addition, as the depth by which the needle 20 has punctured the organ becomes larger, the contact area between the organ and the needle 20 as an electricity flow path becomes larger, and thus the electrical resistance between the needle 20 and the fourth electrode 34 becomes lower. Therefore, by measuring the electrical resistance value between the needle 20 and the fourth electrode 34 when current is caused to flow between the needle 20 and the fourth electrode 34, the depth by which the needle 20 has punctured the organ can be ascertained according to the extent of a change in the value. This aspect is suitably applicable in a case where, for example, the first electrode 31, the second electrode 32, and the third electrode 33 are in contact with the cardiac muscle, and the fourth electrode 34 is not in contact with the cardiac muscle. The electrical resistance can be measured also in a case where each of the first electrode 31, the second electrode 32, and the third electrode 33 is in contact with the cardiac muscle. However, since the cardiac muscle has a higher electrical resistance than blood, the difference in the electrical resistance when the needle 20 has pierced the cardiac muscle from a state of being present in blood is larger so that it is easier to ascertain that the needle 20 has pierced the cardiac muscle, in a case where the electrode is not in contact with the cardiac muscle than in a case where the electrode is in contact with the cardiac muscle. In this manner, improvement of the measurement accuracy can be more facilitated in a state where the electrode is not in contact with the cardiac muscle at all, i.e., in a state where the entirety of the electrode is in contact with blood, than in a state where the electrode is in contact with the cardiac muscle.

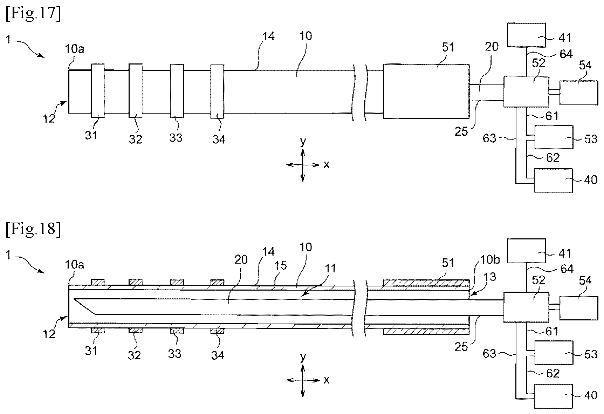

FIG. 17 is a side view showing a modification of the needle system according to the embodiment of the present invention. FIG. 18 is a cross-sectional view (partially a side view) of the needle system shown in FIG. 17.

The second electrode 32 provided to the needle system 1 may be used for detecting an electric signal from the organ. In this case, as shown in FIG. 17 and FIG. 18, the needle system 1 may further include a display device 41 which is connected to the second electrode 32 and which displays the electric signal detected by the second electrode 32. The second electrode 32 may be connected only to the display device 41 or may be connected to each of the display device 41 and the measurement device 40.

The third electrode 33 provided to the needle system 1 may be used for detecting an electric signal from the organ. In this case, as shown in FIG. 17 and FIG. 18, the third electrode 33 is preferably connected to the display device 41. The third electrode 33 may be connected only to the display device 41 or may be connected to each of the display device 41 and the measurement device 40.

The fourth electrode 34 provided to the needle system 1 may be used for detecting an electric signal from the organ. In this case, as shown in FIG. 17 and FIG. 18, the fourth electrode 34 is preferably connected to the display device 41. The fourth electrode 34 may be connected only to the display device 41 or may be connected to each of the display device 41 and the measurement device 40.

As each of the second electrode 32, the third electrode 33, and the fourth electrode 34, an electrode that can detect an electric signal from the organ or an electrode that can detect current caused to flow is preferably used. For example, an electrode that can detect a current intensity, an orientation of a current flow, or the like is preferable. The electric signal includes information such as a current, a voltage, or a potential.

As shown in FIG. 1 to FIG. 3 and FIG. 16, the second electrode 32, the third electrode 33, and the fourth electrode 34 preferably have annular shapes or tubular shapes and are preferably disposed on the distal portion of the resin tube 10.

As shown in FIG. 3, FIG. 4, and FIG. 16, it is preferable to employ a configuration in which: the first electrode 31 and the second electrode 32 are formed in tubular shapes; and the resin tube 10 is disposed in inner cavities of the first electrode 31 and the second electrode 32 formed in tubular shapes. The shape of the first electrode 31 and the shape of the second electrode 32 may be identical to or different from each other. In addition, it is preferable to employ a configuration in which: the third electrode 33 is also formed in a tubular shape; and the resin tube 10 is disposed in an inner cavity of the third electrode 33 formed in a tubular shape. In addition, it is preferable to employ a configuration in which: the fourth electrode 34 is also formed in a tubular shape; and the resin tube 10 is disposed in an inner cavity of the fourth electrode 34 formed in a tubular shape.

The first electrode 31, the second electrode 32, the third electrode 33, and the fourth electrode 34 may be formed from, for example, an electrically conductive material such as platinum or stainless steel. In order to make it easy to ascertain the position of each of the electrodes through radioscopy, the electrode preferably contains a radiopaque material such as platinum. The material forming the first electrode 31, the material forming the second electrode 32, the material forming the third electrode 33, and the material forming the fourth electrode 34 may be identical to or different from one another.

The display device 41 refers to a device that can display an electric signal detected by at least one of the second electrode 32, the third electrode 33, and the fourth electrode 34. Specifically, the display device 41 is preferably a device having a monitoring unit for displaying the electric signal. For example, the monitoring unit of the display device 41 displays, by means of an arrow or a color, a current intensity or an orientation of a current flow detected by at least one of the second electrode 32, the third electrode 33, and the fourth electrode 34 in the heart. A technology for displaying such an electric signal on the display device 41 is generally called 3D mapping. Employment of the above configuration makes it easy for a user to ascertain a to-be-treated portion or a function-losing portion that might cause an arrhythmia. Thus, said employment makes it easy for the user to determine a portion to be punctured by the needle 20.

As shown in FIG. 1 and FIG. 2, a gap of not shorter than a length of one said first electrode 31 or one said second electrode 32 in the longitudinal direction x of the resin tube 10 is preferably present between the first electrode 31 and the second electrode 32.

As shown in FIG. 1 and FIG. 2, a gap of not shorter than a length of one said second electrode 32 or one said third electrode 33 in the longitudinal direction x of the resin tube 10 is preferably present between the second electrode 32 and the third electrode 33.

As shown in FIG. 1 and FIG. 2, a gap of not shorter than a length of one said third electrode 33 or one said fourth electrode 34 in the longitudinal direction x of the resin tube 10 is preferably present between the third electrode 33 and the fourth electrode 34.

As shown in FIG. 1, FIG. 2, FIG. 17, and FIG. 18, the needle system 1 may further include a power supply device 53 which supplies current in order to cause the current to flow between the needle 20 and the first electrode 31. In a case where the second electrode 32 and the measurement device 40 are connected to each other, the power supply device 53 may cause current to flow between the needle 20 and the second electrode 32. In a case where the third electrode 33 and the measurement device 40 are connected to each other, the power supply device 53 may cause current to flow between the needle 20 and the third electrode 33. In a case where the fourth electrode 34 and the measurement device 40 are connected to each other, the power supply device 53 may cause current to flow between the needle 20 and the fourth electrode 34.

Although not shown in detail, a configuration may be employed in which: the needle 20 is connected to a first conductive wire 61; and the first conductive wire 61 is connected to the power supply device 53. In addition, a configuration may be employed in which: the power supply device 53 is connected to a second conductive wire 62; and the second conductive wire 62 is connected to the measurement device 40. A configuration may be employed in which: the first electrode 31 is connected to a third conductive wire 63; and the third conductive wire 63 is connected to the measurement device 40. In a case where the second electrode 32, the third electrode 33, and the fourth electrode 34 are connected to the measurement device 40, a configuration may be employed in which: a plurality of the third conductive wires 63 are provided; the second electrode 32, the third electrode 33, and the fourth electrode 34 are connected to respective independent ones of the third conductive wires 63 different from the third conductive wire 63 to which the first electrode 31 is connected; and each of the third conductive wires 63 is connected to the measurement device 40. That is, each of the electrodes is connected to the measurement device 40 via an independent one of the conductive wires.

Although not shown in detail, in a case where the second electrode 32, the third electrode 33, and the fourth electrode 34 are connected to the display device 41, a configuration may be employed in which: a plurality of fourth conductive wires 64 are provided; the second electrode 32, the third electrode 33, and the fourth electrode 34 are connected to respective independent ones of the fourth conductive wires 64; and each of the fourth conductive wires 64 is connected to the display device 41. That is, each of the electrodes is connected to the display device 41 via an independent one of the conductive wires.

This application claims the benefit of the priority date of Japanese patent application No. 2022-172403 filed on October 27, 2022. All of the contents of the Japanese patent application No. 2022-172403 filed on October 27, 2022 are incorporated by reference herein.

### REFERENCE SIGNS LIST

1: needle system
10: resin tube
11: inner cavity of the resin tube
12: distal-side opening
13: proximal-side opening
14: outer surface of the resin tube
15: inner surface of the resin tube
20: needle
21: inner cavity of the needle
22: opening
23: side wall
24: hole
25: outer surface of the needle
26: inner surface of the needle
31: first electrode
32: second electrode
33: third electrode
34: fourth electrode
40: measurement device
41: display device
51: first handle
52: second handle
53: power supply device
54: liquid supply device
61: first conductive wire
62: second conductive wire
63: third conductive wire
64: fourth conductive wire
71: first insulating base material
72: second insulating base material
74: hole

## Claims

1. A needle system comprising:
a resin tube having an inner cavity extending in a longitudinal direction of the resin tube;
a needle formed from an electrically conductive material and disposed in the inner cavity of the resin tube so as to be movable in the longitudinal direction with respect to the resin tube, the needle being configured to puncture an organ inside a body;
a first electrode disposed on an outer surface of the resin tube; and
a measurement device configured to measure an electrical resistance between the needle and the first electrode when current is caused to flow between the needle and the first electrode.

2. The needle system according to claim 1, wherein the first electrode has an annular shape and is disposed on a distal portion of the resin tube.

3. The needle system according to claim 1 or 2, wherein
the needle system further comprises a second electrode disposed on the outer surface of the resin tube so as to be located on a proximal side relative to the first electrode, and
the measurement device is configured to measure an electrical resistance between the needle and the second electrode when current is caused to flow between the needle and the second electrode.

4. The needle system according to claim 3, wherein a gap of not shorter than a length of one said first electrode or one said second electrode in the longitudinal direction is present between the first electrode and the second electrode.

5. The needle system according to claim 1, wherein the needle has an inner cavity extending in the longitudinal direction.

6. The needle system according to claim 5, wherein the needle has, in a distal end thereof, an opening in communication with the inner cavity of the needle.

7. The needle system according to claim 5 or 6, wherein
the needle has a side wall defining the inner cavity of the needle, and
a hole in communication with the inner cavity of the needle is formed in the side wall of the needle within a distal portion of the needle.

8. The needle system according to claim 7, wherein
a plurality of the holes are formed in the side wall of the needle, and
the plurality of the holes are arranged in the longitudinal direction.

9. The needle system according to claim 1 or 2, wherein the needle is, over an entirety thereof, formed from a metal.

10. The needle system according to claim 1 or 2, wherein a first insulating base material is disposed on an outer surface, of the needle, that includes a distal end of the needle.

11. The needle system according to claim 10, wherein a second insulating base material is disposed on the outer surface of the needle so as to be located on a proximal side relative to a proximal end of the first insulating base material.
